(19) Europäisches Patentamt
European Patent Office
Office européen des brevets

(11) **EP 2 492 691 A1**

(12) **EUROPEAN PATENT APPLICATION**

(43) Date of publication:
**29.08.2012 Bulletin 2012/35**

(51) Int Cl.:
*G01N 33/68* [(2006.01)]   *C12Q 1/68* [(2006.01)]

(21) Application number: **11382047.6**

(22) Date of filing: **22.02.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Institut de Recerca Hospital Universitari Vall d'Hebron 08035 Barcelona (ES)**

(72) Inventors:
• **Montaner Villalonga, Joan**
  **08037, Barcelona (ES)**
• **Rosell Novel, Anna**
  **08041 Barcelona (ES)**
• **Navarro Sobrino, Miriam**
  **08013, Barcelona (ES)**

(74) Representative: **Carvajal y Urquijo, Isabel et al Clarke, Modet & Co.
c/ Goya, 11
28001 Madrid (ES)**

(54)   **Method of predicting the evolution of a patient suffering of a neurovascular disease**

(57)   The invention is a method of predicting the evolution of a patient suffering of a neurovascular disease; preferably stroke, comprising obtaining a biological sample of said patient, assessing in said biological sample the level of endostatin and FasL, determinining whether said levels of endostatin and FasL together are above or below predetermined cut-off levels and predicting the functional outcome of said neurovascular disease on said patient evaluating the result of the previoues step. The combination of endostatin and FasL is a more powerful predictor that any clinical prognostic tool and adds significant prognostic value to all other clinical variables of the art.

FIG. 3

**Description**

**FIELD OF THE INVENTION**

The present invention describes a method for determining the outcome of a patient suffering of stroke measuring two biological markers combined, endostatin and FasL. The combination of biomarkers of the invention is revealed useful for all kinds of stroke. The invention is of appliance in the clinic phase at the diagnosis of the evolution of patients suffering of all kinds of neurovascular diseases.

**BACKGROUND ART**

Stroke is the third leading cause of death and the most common cause of permanent disability in adults worldwide. When stroke happens, the medical reaction time is of greatest importance for the patient as neurological deterioration may continue after the acute phase. To acknowledge the mechanisms of occurencé of stroke worsening after the acute phase is crucial, and the maximal information relating patient evolution is needed at the decission of the right terapeutic measures that may help to avoid permanent neurological deficits or dead.

The use of plasma biomarkers is getting increasingly popular in several fields of medicine. Decision making processes using biomarkers is widely accepted in medical situations such as initiating lipid lowering therapies (LDL), diagnosing acute myocardial infarction (troponins), ruling-out pulmonary embolism suspicions (D-dimer), etc. At cerebrovascular diseases is also interesting, since biomarkers might help physicians in several steps of stroke evaluation. Injury-related pathological pathways have been the main searched focus for potential biomarkers (Shenhar-Tsarfaty et al. "Interleukin-6 as an early predictor for one-year survival following an ischaemic stroke/transient ischaemic attack". Int J Stroke. 2010 Feb;5(1):16-20).

Advances in the management of stroke patients in the last years, including new therapeutic and diagnostic strategies, allowed to reduce the in-hospital lethality and to achieve better functional outcomes. Even at neurological ward it might be interesting to save days, due to the high cost. Early discharge might avoid consuming a lot of resources if it is done safely with high prediction on the worsening of patients, or on complications after discharge.

Since the establishment of cost-effective medical care for stroke is of a great importance, studies are needed in the technique to assess the cost-effectiveness and cost-benefit ratio of different stroke care concepts. In relation to this, biomarkers that might predict outcome do aid in the allocation process of the treatment of patients.

Several biomarkers have been shown to be useful at neurological prognosis such as worsening following ischemic stroke, for example IL-6, ICAM-1 or TNF-alfa ("Blood markers for the prognosis of ischemic stroke: a systematic review". Whiteley W, et al. Stroke. 2009 May;40(5):e380-9). Stroke is an etiologically heterogeneous disease. Identification of the specific cause in every patient has important clinical implications. Brain natriuretic peptide (BNP) and D-dimer have been reported as specific markers of cardioembolic stroke. Moreover, increased S100B level was also found after acute spontaneous intracerebral hemorrhage ("Etiologic diagnosis of ischemic stroke subtypes with plasma, biomarkers". Montaner J et al. Stroke. 2008 Aug;39(8):2280-7). All these biomarkers were also associated with a worse early and late evolution in stroke patients, however not as accurate of the combination of the present invention.

Many of the biomarkers relating stroke are linked to neuroinflammatory cascades. The inventors recently showed that admission levels (< 3h of symptoms onset) of high sensitivity C-Reactive Protein (CRP) predicts mortality after tissue-Plasminogen Activator (t-PA) treatment for stroke patients, adding prognostic information to classical risk factors. This is of clinical relevance because CRP is a powerful marker that might be used as a point-of-care tool for the risk stratification of stroke patients' candidates to receive t-PA. Together with high CRP, advanced age is another independent predictors of mortality following t-PA. The risk of death following t-PA reached 54% in patients older than 72 years who were in the upper CRP quartile. Therefore both factors (age and CRP) might negatively interact.

Malignant Middle Cerebral Artery (MCA) infarction shows very bad prognosis and huge mortality rates. Serena et al. conducted a study to determine whether molecular markers of endothelial damage may help to predict secondary brain edema and to identify patients who could benefit from aggressive therapies such as decompressive hemicraniectomy or hypothermia (Serena J, et al. "The prediction of malignant cerebral infarction by molecular brain barrier disruption markers." Stroke. 2005 Sep;36(9):1921-6). They showed that c-Fn> 16.6 microg/mL had the highest sensitivity (90%), specificity (100%), and negative and positive predictive values (89% and 100%, respectively) for the prediction of malignant MCA infarction.

Although thrombolytic therapy in acute stroke is effective since it accelerates clot lyses and earlier restoration of blood flow, up to 40-50% of treated patients do not recanalyze or do it too late, and between 6 and 15% suffer hemorrhagic transformations (HT) with high death rates. In humans, biomarkers such as MMP-9 or fibronectin, which might be used to select patients at higher risk of hemorrhagic Transformation (HT), and high PAI-1 interfering with tPA-induced recanalization, thus predicting clot-lyses resistance and poor outcome, have been recently identified (Montaner J. "Blood biomarkers to guide stroke thrombolysis". Front Biosci (Elite Ed). 2009 Jun 1;1:200-8).

One of the main limitations of most stroke related biomarkers is that they do not render as independent predictors of outcome. Are these biomarkers included in multivariate models together with else clinical variables such as age or NIHSS, results show that they do not render as independent predictors any more, adding very few or no prediction value to the well known clinical variables. NIHSS reflects the initial severity of the stroke. The incorporation of inflammatory marker levels to validated prognostic models do not effectively improve model discrimination, calibration, or reclassification in the prediction of outcome after stroke.

Fas ligand (FasL) is a type II transmembrane protein of the Tumor Necrosis Factor (TNF) family, able to induce apoptosis when bound to its receptor, Fas. The Fas system is an apoptosis-signalling receptor located on the cell surface that belongs to the tumor necrosis factor receptor family. The interaction between Fas and its ligand FasL leads to apoptosis. The soluble form of the anti-apoptotic regulator Fas (sFas) and the pro-apoptotic factors soluble FasL (sFasL) are able to be measured. Following brain bleedings, sFas, which is thought to inhibit Fas interaction with its ligand, was decreased within the first 24 hours after intracerebral hemorrhage. It was also decreased in cerebrospinal fluid from ischemic stroke patients and was inversely correlated with brain infarct volume and neurologic deficit 3 weeks and 3 months after the event (Tarkowski E, et al. Intrathecal expression of proteins regulating apoptosis in acute stroke. Stroke. 1999 Feb;30(2):321-7).

FasL has been related with cancer as an immune system modulator; however few with stroke. In this sense, Delgado suggests the existence of Fas-mediated apoptosis involvement after Intracerebral Hemorrhage (ICH) in perihematomal areas (Delgado et al. "Fas system activation in perihematomal areas after spontaneous intracerebral hemorrhage" Stroke. 2008;39:1730-1734). He describes that FasL mediates in post-stroke apoptosis.

WO 2010031821 describes FasL as marker for a differential diagnosis to determine whether a patient has suffered of stroke vs. any other pathology mimicking a stroke. However, FasL alone shows a poor mRS correlation to be clinically relevant, which makes it not particularly suggested to be used in combined diagnose with respect to other biomarkers of the art.

Endostatin is a protein derived from collagen XVIII, which is a major proteoglycan of both endothelial and epithelial basement membrane zones. An elevated circulating-endostatin level has been found in several disease, such as diabetic retinopathy, systemic sclerosis, acute lung injury, malignant glioblastomas and coronary artery disease. It shows measurable levels in blood, and works as an angiogenesis inhibitor factor: it inhibits the build up of new blood vessels. The action of Endostatin has been further directed to oncologic topics. WO 2009033406 A1 among others, propose its use in the treatment of tumors. Other studies relate the antiangiogenic use of endostatin for treating ocular neovascularization.

The inventors have demonstrated that a predominance of endostatin over Vascular Endothelial Growth Factor (VEGF) within the endogenous angiogenic response is associated with larger extent and risk of recurrence of symptomatic intracranial atherosclerosis (Arenillas et al. Angiogenesis in symptomatic intracranial atherosclerosis: predominance of the inhibitor endostatin is related to a greater extent and risk of recurrence. Stroke. 2005 Jan;36(1):92-7). Besides, they reported that tPA-treated ischemic stroke patients showing higher endostatin level had an impaired functional outcome, meaning that these patients were dependent in activities of daily living three months after stroke.

Tian identifies high levels of endostatin in rabbit ischemic tissue (Tian et al. "Increased protein and mRNA expression of endostatin in the ischemic brain tissue of rabbits after middle cerebral artery occlusion". Neurosci Bull. 2007. 23(1): 35-40). They conclude that cerebral ischemia leads to an up-regulation of endostatin in the brain.

Rosell teaches that the endostatin level is inversely related to the clinical improvement evolution of patients suffering of stoke (Montaner et al. "High level of an Angiogenesis Inhibitor (endostatin) is Related to poor Outcome in Ischemic Stroke Patients treated with t-PA. 2005 www.tesisenxarxa.net), which is considered the closest publication of the art to the present invention. It is the first study showing that an angiostatic factor, plasma endostatin, is over-expressed after acute cardioembolic stroke. The publication shows positive correlation between endostatin and the modified Rankin Scale score (mRS, ranges patients of stroke by functional outcome from 0="no symptoms" to 5="very severe discapacity" and 6="dead"), relating plasmatic endostatin levels during the acute phase with further evolution of the pathology. Thus, they indentify the role of endostatin as a biologic marker of the evolution of stroke in a patient. However, endostatin alone shows poor mRS correlation (r=0.357; p=0.035). This marker considered alone does not provide more accurate information than any other classical marker, for example NIHSS or the simple age of the patient. Therefore, the use of endostatin in combined diagnose cannot be considered particularly suggested with respect to other biomarkers of the art.

In the present application "r" is meant as the correlation coefficient, referring to a number between minus and plus one (-1.0 to 1.0) that measures the amount of agreement between two variables, meaning how close the graph drawn linking to these two variables is to a straight line.

In the present application "**P value**" is meant as the probability (ranging from zero to one) of some results to have occurred by chance if the null hypothesis of no effect was true. A value of p $\leq$0.05 is often used as a threshold to indicate statistical significance.

Recently, several panels of biomarkers have been proposed to be used in the diagnosis of ischemic stroke (Blood biomarkers in acute stroke. Foerch C, et al. Neurology. 2009 Aug 4;73(5):393-9). The international application WO 2004/059293 A2 teaches about a panel of biomarkers for the differential diagnosis of stroke including the matrix metalloproteinase 9, brain natriuretic factor, d-dimer and protein S100beta (Clinical usefulness of a biomarker-based diagnostic test for acute stroke:

the Biomarker Rapid Assessment in Ischemic Injury (BRAIN) study. Laskowitz DT, et al. Stroke: 2009 Jan;40(1): 77-85). However, after the knowledge of the inventors, no panel or combination of biomarkers is being used in the daily practice to predict stroke outcome.

The problem of the art is then to find a suitable marker indicative of the clinical evolution of a patient suffering of neurovascular diseases, showing higher mRS correlation than those at disposal in the art. The solution proposed by the present invention is the use of endostatin and FasL combined, which surprisingly show a synergistic diagnose action in stroke and result in better prediction accuracy than any other marker used in the art, often correcting their results.

## SUMMARY OF THE INVENTION

The present invention is a method of predicting the evolution of a patient suffering of a neurovascular disease comprising obtaining a biological sample of said patient, assessing in said biological sample the level of endostatin and FasL or of respective nucleic acid molecules encoding same, determinining whether said levels of endostatin and FasL together, or of respective nucleic acid molecules encoding same, are above or below predetermined cut-off levels and predicting the functional outcome of said neurovascular disease on said patient evaluating the result of the previous step.
In the scope of the present application, the obtention of a biological sample of the patient does not comprise any surgical step that could involve substantial risk for the health of the said patient. Further, the evolution of the patient is directly related to the functional outcome of the disease.
A referred embodiment of the present invention is that said neurovascular disease is stroke, and a more preferable embodiment is that said biological sample has been taken out from the patient along the next 24 hours after an acute episode of stroke.
The present application shows that those stroke patients functionally dependent at three months of the ischemic event had a higher level of endostatin and FasL in the hyperacute phase, when arriving to the emergency department. The inventors demonstrate the positive correlation of the systemic levels of these two markers, endostatin and FasL, with the evolution of patients of stroke. These plasmatic markers measured right after the onset of stroke, are able to predict with great specificity the functional prognosis of the patient. This makes the combination of the present invention a potent and very specific marker of the evolution of said patients.
The marker combination of the invention is applicable to any neurovascular disease. The same or family-related biological markers and clinical parameters are used in the diagnose of the entire family of neurovascular diseases, which also share similar neurological symptoms such as the occlusion of small or large cerebral arteries leading to brain ischemia or the rupture of vessels, leading to brain hemorrhages. Therefore, the combination of endostatin and FasL should be accurate for all stroke related diseases, the invention not being restricted to stroke.
In the scope of the present application, the term "biological marker" or "biomarker" refers to any measurable parameter (anatomic, physiologic, biochemical or molecular) able to monitor a physiological or pathological process such as the onset of disease, the response to medical intervention or relapse, or the body's response to external environmental factors. A "prognostic biomarker" is a measurable parameter incorporated into a validated test that renders predictive of the progression of the disease in an individual case, whereas a "diagnostic biomarker" is predictive of the disease onset.
A preferred embodiment of the invention is that the biological sample taken from the patient is a blood sample. Another embodiment is that said biological sample is a plasma sample. Another embodiment is to be a serum sample, and another one is a saliva sample.
The invention further exemplifies particular cut-off levels for both markers considered together, from which it can be established that patients have 100% posibilities of a "dependent'" functional status evaluated with respect to the ranking score at the third month. The specificity value of the prognosis from the cut-off levels is, then, 100%. In the context of the provided examples, any patient that exceeded of 184 ng/ml of endostatin and 37.1 pg/ml of FasL had 100% possibilities of worsening their prognose.
In the scope of the present application, a "dependent rankin" mRS>2, refers to a bad functional prognosis. The "modified Rankin Scale" (mRS) is a scale accepted in the art for measuring the degree of disability or dependence in the daily activities of people who have suffered a stroke. It has become the most widely used clinical outcome measure and end-point for most stroke clinical trials. The scale runs from 0-6, from perfect health without symptoms up to death.

- 0 - No symptoms.

- 1 - No significant disability. Able to carry out all usual activities, despite some symptoms.

- 2 - Slight disability. Able to look after own affairs without assistance, but unable to carry out all, previous activities.

- 3 - Moderate disability. Requires some help, but able to walk unassisted.

- 4 - Moderately severe disability. Unable to attend to own bodily needs without assistance, and unable to walk unassisted.

- 5 - Severe disability. Requires constant nursing care and attention, bedridden, incontinent.

- 6-Dead.

Patients with high levels of endostatin at baseline (< 3h after symptom onset) will be dependent at three months, meaning they will not be able to do their daily activities independently.

The invention shows that mRS-scored ≤2 patients (independent patients) arrived to the emergency department with low combined levels of endostatin and FasL and developed towards a good evolution. On the other side, high levels of these markers measured in the acute phase of stroke would point to a less favourable evolution. When evaluating functional outcome at three months, inventors found that dependent patients, mRS> 2, patients with bad outcome of the disease, had had higher level of both biomarkers (p< 0.05). The combination of endostatin and FasL levels reaches 100% of specificity. The higher specificity of a prognosis biomarker is determinant to assure that all patients turning to be dependent in three months do receive the most accurate medical attendance. Only the novel combination of the biomarkers of the invention shows the highest predictive value.

Possible combinations of independent diagnostic stroke markers do not usually show any added or more accurate prognosis. However, the combined test of endostatin and FasL is more specific than using each one of them separately; moreover the combined test of endostatin and FasL is more powerful predictor that any clinical prognostic tool and adds significant prognostic value to all other clinical variables known to be associated with poor outcome. The combination of the invention shows a surprising diagnose synergistic effect, which confers it with inventive activity over the technique.

An embodiment of the method of the present invention comprises endostatin and FasL in combination with at least another biomarker for the prediction of the evolution of a patient suffering of a neurovascular disease. Another embodiment is the method of the invention comprising endostatin and FasL in combination with at least one clinical parameter for the prediction of the evolution of a patient suffering of a neurovascular disease, preferably NIHSS, age or doppler-tested artery degradation.

A further embodiment of the method of the invention is, that prediction of the evolution of the patient suffering of a neurovascular disease enables a physician to determine the degree and type of therapy recommended to prevent or treat said neurovascular disease.

Still another embodiment of the invention is the use of endostatin and FasL combined as a predictor of the evolution of a patient suffering of a neurovascular disease, preferably when the levels of endostatin and FasL of said combination above certain cut-off levels are searched in a biological sample.

A preferred embodiment of the present invention is a kit for predicting the evolution of a patient suffering of a neurovascular disease, preferably stroke, comprising appropriate reagents for the quantification of endostatine and FasL in a biological sample of a patient suffering of a neurovascular disease, and enabling the comparison of the combined result of said quantification with predermined cut-off values. Another preferred embodiment is a kit including at least one container that contains appropriate pharmaceutical formulations for the quantification of endostatin and FasL in a biological sample of a patient suffering of a neurovascular disease, preferably stroke, instructions for the use of said formulations and a dispositive for determining whether the result of the quantification of endostatin and FasL combined are above or below predetermined cut-off levels indicative of the likehood of a prognosis of functional outcome of the neurovascular disease in said patient. Preferably, the tested biological sample is selected among a blood sample, a plasma sample, a serum sample and a saliva sample.

Another embodiment of the invention refers to a method of predicting the clinical evolution of a patient suffering of stroke related to the level of endostatin and FasL, or to the levels of nucleic acid molecules encoding the endostatin and FasL proteins, said method comprising the step of detecting in a suitable biological sample of said patient the level of endostatin and FasL proteins.

The invention offers the possibility of managing the diagnose prognosis of a wide number of patients in else centers than those wherein the biological samples are obtained. A further embodiment of the invention is, then, a system for the prediction of the evolution of a patient of a neurovascular disease, preferably stroke, comprising data processing means, said data processing means been configured to assess in said biological sample the level of endostatin and FasL, or of respective nucleic acid molecules encoding same; to determine whether said levels of endostatin and FasL together or of respective nucleic acid molecules encoding same, are above or below predetermined cut-off levels; and to predict the functional outcome of the neurovascular disease of the patient evaluating the result of the previous determination.

**BRIEF DESCRIPTION OF THE FIGURES**

**Figure 1.** Receiver Operating Characteristic (ROC) curves of baseline FasL level'(A) and baseline endostatin (B). Arrows indicates the cut-off points for FasL (37.1 ng/ml) and endostatin (184 ng/ml) with the highest sensitivity and specificity to identify dependency at three months.

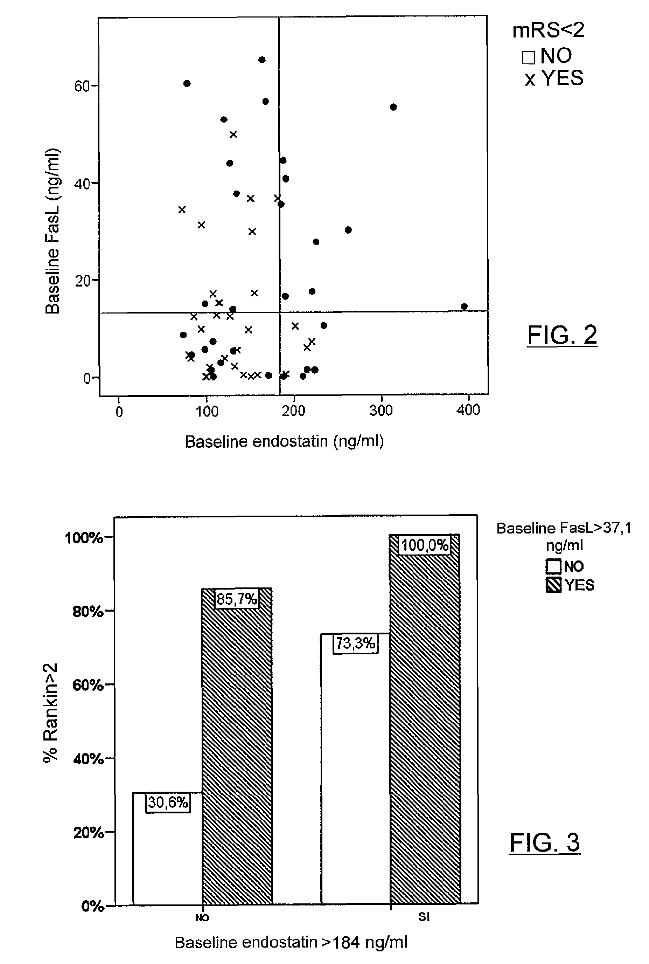

**Figure 2.** Endostatin (X-axis) and FasL (Y-axis) levels in dependent (mRS> 2) stroke patients (black dots) and independent (mRS≤2) stroke patients (black crosses). The lines are traced through the previously selected cut-offs for both biomarkers and show that only patients with bad functional outcome (black dots) are located in the area with high blood levels for both markers (upper-right quadrant).

**Figure 3.** Percent of stroke dependent patients (rankin> 2) depending on endostatin level (equal to or higher than 184 ng/ml) and FasL (equal to or higher than 37.1 ng/ml).

**Figure 4.** ROC curves of clinical variable , model and clinical variable model+ biomarkers compared to reference line.

In the scope of the present description, "clinical variable" is meant to demographic data indicative of stroke such as age, gender and familial history of stroke, as well as to clinical information such as the presence of hypertension, dyslipidemia, diabetes, atrial fibrillation, cardiac disease, smoking, medication, and past clinical history of stroke.

**DETAILED DESCRIPTION OF PARTICULAR EMBODIMENTS**

The following examples are included as support of the claimed subject matter, in no case limiting the scope of the present invention.

**Example 1: Selection of patients**

The study included patients with an acute ischemic stroke (n= 61) involving the Middle Cerebral Artery (MCA) territory, admitted in the emergency department of Hospital Vall d'Hebron, Barcelona, Spain, All patients received thrombolytic therapy in a standard dose of 0,9 mg/Kg (10% bolus and 90% continuous infusion for 1 h) within 3h of symptoms onset. At admission, all patients underwent a Cranial computed Tomography (CT) scan and a second CT scan was repeated 24 to 48h later (or earlier when rapid neurological deterioration occurred) to evaluate the presence and type of Hemorrhagic Transformation (HT), that was defined according to previously published criteria (Hacke et al., Thrombolysis with alteplase 3 to 4.5 hours after acute ischemic stroke. N Engl J Med. 2008; 359:1317-29, Pessin 1990). Transcranial Doppler (TCD) measurements were performed by an experienced neurologist, blinded for laboratory results, with the use of a Multi-Dop X4 TCD (DWL Elektroniche System GmbH) device. TCD examinations were performed before the beginning of the treatment, at the end of t-PA infusion and serially for the first 24h. Proximal or distal MCA occlusions and follow-up recanalization degrees were defined as previously described (Burgin et al, Transcranial doppler ultrasound criteria for recanalization after thrombolysis for middle cerebral artery stroke. Stroke 2000; 31:1128-32).

A detailed history of vascular risk factors, drug abuse, alcoholism and concomitant medication was obtained from each patient. National Institute of Health Stroke Scale Scores (NIHSS) were recorded to assess level of consciousness and necrological status at admission and during follow-up visits (2, 12, 24, 48h and at discharge between 3-7 days). Neurological improvement was defined as a decrease in NIHSS≥4 points and neurological deterioration as either death or increase in NIHSS≥4 points at 48h. Functional outcome was defined by modified Rankin Scale (mRS) three months after stroke. Patients were considered dependent when rankin scale score was > 2 points.

Venous blood samples were drawn from each patient at admission (baseline). Plasma was extracted in EDTA tubes, immediately separated by centrifugation at 3,000 rpm for 15 minutes and stored at -80°C until analysis. Plasma level of human endostatin was determined by commercially available ELISA (Quantikine, R& D Systems, MN, USA) and FasL through SearchLight technology. Each plasma sample was analyzed twice and the mean of, two values was given in ng/mL. The mean intra-assay coefficients of variation were less 30% for both biomarkers. Endostatin and FasL levels were expressed as mean± standard desviation. Statistical significance for intergroup differences was assessed by Mann-Whitney test. P<0.05 was considered statistically significant. To study correlations between continuous variables, Spearman coefficients were used. Endostatin and FasL level did not follow a normal distribution (Kolmogorov-Smirnov and P-P plot). Endostatin and FasL (mean± standard desviation) levels and their association with demographic characteristics, clinical variables and risk factors profile in stroke patients depending the demographic caractheristics is shown in Table 1.

**Table 1**

| Endostatin | | | |
|---|---|---|---|
| | levels | | |
| | Yes | No | p-value |
| **Sex (women)** | 169.73±66.36 | 135.06±51.74 | 0.026* |
| **HTA** | 156.16±69.81 | 144.89±46.62 | 0.484 |
| **Tobacco** | 119.06±31.44 | 161.74±65.19 | 0.03* |
| **Atrial Fibrilation** | 175.64±69.69 | 136.96±51.02 | 0.015* |
| **Diuretics** | 179.26±76.99 | 136:94±44.41 | 0.053 |
| **Parenchymal Hematoma** | 195.13±43.41 | 145.9±61.16 | 0.044* |
| **mRS>2** | 169.81±72.34 | 132.67±40.07 | 0.016* |
| **Vessel (MCA)** | 154.1±61.19 | 122.95±68.79 | 0.558. |
| **TCD b (proximal)** | 152.8±70.1 | 150.01±49.55 | 0.867 |
| **TCD 12h (proximal)** | 100.54±23.5 | 157.88±40 | 0.018* |
| **TOAST (athero)** | 147.51±70.5 | 152.74±58.92 | 0.046* |

| Fas L | | | |
|---|---|---|---|
| | levels | | |
| | Yes | No | p-value |
| **Sex (women)** | 21:32±19.57 | 13.82±16.52 | 0.072 |
| **HTA** | 14.3±17,6 | 21.83±18.67 | 0.085 |
| **Tobacco** | 15.64±17.69 | 19.43±18.8.2 | 0.386 |
| **Atrial Fibrillation** | 18.43±18.53 | 16.76±18.34 | 0.508 |
| **Diuretics** | 15.93±15.24 | 17.55±18.95 | 0.776 |
| **Parenchymal Hematoma** | 25.8±15.71 | 16.29±18.43 | 0.154 |
| **mRS>2** | 21.77±21.3 | 12.85±13.42 | 0.17 |
| **Vessel (MCA)** | 15.88±17.6.2 | 34.24±20.83 | 0.063 |
| **TCD b (proximal)** | 20.25±19.88 | 10.48±11.3 | 0.081 |
| **TCD 12h (proximal)** | 8.6±4.5 | 7.8±4.5 | 0.08 |
| **TOAST (athero)** | 16.3±20.38 | 17.7±17.83 | 0.579 |

HTA: Arterial hypertension; mRS: modified Rankin scale; TCD: Transcranial Doppler examinations, TOAST: The Trial of Org 10172 in Acute Stroke Treatment, Athero: atherotrombotic etiology. * p value ≤0.05.

The correlation between endostatin and FasL levels and age and stroke severity in stroke patients is shown in Table 2.

**Table 2**

| | Endostatin | | Fas L | |
|---|---|---|---|---|
| | R² | p-value | R² | p-value |
| **Age** | 0.4 | 0.001 * | 0.889 | 0.018* |
| **NIHSS Basal** | -0.97 | 0.005* | 0.302 | 0.018* |
| **NiHSS 1h** | 0.993 | 0.001* | 0.324 | 0.015* |
| **NIHSS 2h** | 0.528 | 0.089 | 0.337 | 0.014* |
| **NIHSS 24h** | 0.879 | 0.02* | 0.266 | 0.038* |
| **NIHSS 48h** | 0.219 | 0.166 | 0.268 | 0.044* |

(continued)

| | Endostatin | | Fas L | |
|---|---|---|---|---|
| | R² | p-value | R² | p-value |
| **NIHSS alta** | 0.367 | 0.007* | 0.213 | 0.127 |

R²: Correlation coefficient between biomarker levels and age or stroke severity.
*p value ≤0.05, NIHSS: National Institute of Health-Stroke Scale Scores.

## Example 2: Determination of cut-off values for endostatin and FasL

Different demographic, clinical variables and risk factors were associated with good (mRS ≤2) and poor outcome (mRS> 2) in stroke population. Table 3 shows the clinical, demographic characteristic and biomarker levels associated to stroke functional outcome (mRs < or > 2) at three months and shows the analysis of factors influencing stroke functional outcome together with those biomarkers. The analysis of factors influencing stroke functional outcome (mRS> 2) was assessed by Mann-Whitney test.

**Table 3**

| | Global | Rank<2 | rank>2 | P-value |
|---|---|---|---|---|
| **Age** | 70.44±12.6 (36-94) | 68.13±13.3 | 72.68±11.7 | 0.097 |
| **Sex (women)** | 29 (47.5%) | 46.70% | 48.40% | 0.893 |
| **Tobacco** | 11 (19.6%) | 28.60% | 10.7% | 0.093 |
| **HTA** | 36 (59%) | 56.70% | 61.30% | 0.714 |
| **DM** | 15 (24.6%) | 20.00% | 29.00% | 0.413 |
| **ACxFA** | 23 (37.7%) | 26.70% | 48.40% | 0.008* |
| **Ischemic cardiopathy** | 11 (13%) | 16.70% | 19.40% | 0.785 |
| **Dyslipemia** | 15 (25%) | 23.30% | 26.70% | 0.766 |
| **Previous stroke** | 9 (14.8%) | 10.00% | 19.40% | 0.303 |
| **Aplatelet** | 20 (33.3%) | 27.60% | 38.70% | 0.361 |
| **Statin** | 13 (21.3%) | 20% | 22.60% | 0.806 |
| **ADO** | 12 (21.8%) | 17.20% | 26.90% | 0.385 |
| **DIUASA** | 25 (42.4%) | 34.50% | 50.00% | 0.228 |
| **ARA II** | 4 (6.8%) | 6.90% | 6.70% | 0.972 |
| **ACE-I** | 11 (18.6%) | 17.20% | 20.00% | 0.786 |
| **Fas L >37.1** | 10 (16.4%) | 3.30% | 29% | 0.007* |
| **Endostatin >184** | 1 (29.5%) | 13.30% | 45.20% | 0.006* |
| **TCD b (proximal)** | 35 (59.3%) | 40% | 79.30% | 0.002* |
| **TCD 1h** | 23 (41.1%) | 17.20% | 66.70% | 0.001* |
| **TCD 2h** | 15 (32.6%) | 4.50% | 58.30% | <0.001* |
| **TCD 24h** | 3(11.1%) | 0% | 25% | 0.031 * |
| **Evolution 48h** | 38 (62.3%) | 90% | 35.50% | <0.001* |
| **NIHSS b** | 16 (11-20) | 14 (9.75-18.5) | 17 (10.25-19.75) | 0.035* |
| **NIHSS 1h** | 14.5 (9-19) | 11 (8.25-16.25) | 17 (9.5-17) | 0.013* |
| **NIHSS 2h** | 11 (7.5-17) | 9 (5-11) | 16.5 (8.25-16.5) | 0.002* |
| **NIHSS 12h** | 9.5 (5-18.25) | 6 (4-10) | 15.5 (8.25-19.75) | <0.001* |
| **NIHSS 24h** | 8(4-16.5) | 5 (2-9.25) | 15.5 (8.25-18.75) | <0.001* |
| **NIHSS 48h** | 7 (2-16) | 2.5 (1.75-7) | 15.5 (10.25-17.75) | <0.001* |
| **NIHSS alta** | 3 (1-10) | 2 (1-4) | 13.5 (6.25-20.5) | <0.001* |

HTA: Hypertension; DM: Diabetes mellitus; ACxFA: Atrial fibrillation, ADO: Oral antidiabetic drugs; DIUASA: antidiuretic drug; ARA II: Angiotensin II receptor antagonism ; ACE: Angiotensin-converting enzyme I; TCD: Transcranial Doppler examinations; NIHSS: National Institute of Health Stroke Scale Scores. * P< 0.05 was considered statistically significant.

To determine the endostatin and FasL cutt-off values Receiver Operator Characteristics (ROC) curves were performed. A ROC curve, is the graphical plot of the sensitivity, or true positive rate, vs. false positive rate (1 - specificity or 1 - true negative rate). In the scope of the present application, the term "sensitivity" refered to the percentage of the proportion of actual positives which were correctly identified as such; e.g. the percentage of sick people who was correctly identified as having the condition. The term "specificity" measures the proportion of negatives which were correctly identified; e.g. the percentage of healthy people who was correctly identified as not had the condition.

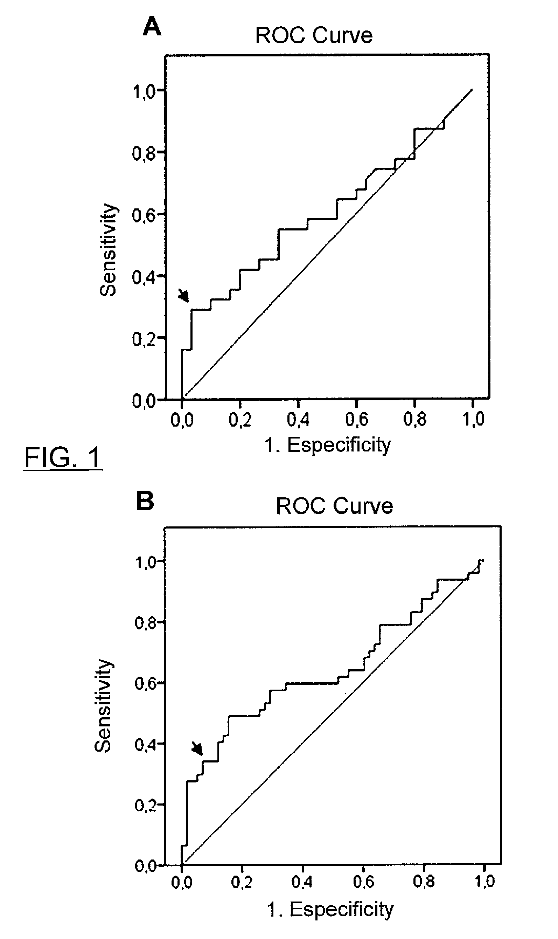

A theoretical optimal prediction must achieve a 100% sensitivity, i.e. predict all people from the sick group as sick, and a 100% specificity i.e. not predict anyone from the healthy group as sick. To identify the best cut-off values a ROC curve was performed for each biomarker and using that values a logistic regression was performed with other parameters related with outcome to determine factors that could be considered as independent predictors of dependency at three months (mRS>2; dependency), using the forward step-wise method by the likelihood ratio test (Figure 1). A 184 ng/ml endostatin cut-off point had 45.2% sensitivity and 86.7% specificity to identify dependency at three months was detected and FasL a 37,1 ng/ml cut-off point had 29% sensitivity and a 96.7% specificity to detect dependency at three months.

### Example 3: Sensitivity of endostatin and FasL

To calculate the sensitivity and specificity for biomarker cut-off values to predict stroke outcome, a receiver operator characteristic (ROC) curve was configured to establish the cut-point of endostatin and FasL with the optimal sensitivity and specificity predicting unfavourable outcome (mRS< 2). Selected biomarkers were modelled as dichotomous variables (with the cut-off values obtained from the ROC curves), and other variables such as NIHSS score, baseline proximal occlusion and age were included as continuous variables in different logistic models. Finally, three logistic regression analyses were performed to determine the factors that could be considered as independent predictors of unfavourable outcome using the forward stepwise method by the likelihood ratio test. An equation from the regression model was performed to calculate the predictive probability of dependency at three months. A p value < 0.05 was considered significant.

A 184 ng/ml endostatin cut-off point had 45.2% sensitivity and 86.7% specificity to identify dependency at three months of the ischemic event with a positive predictive value -of 77.8% and a negative predictive value of 60.5%. Regarding FasL a 37,1 ng/ml cut-off point had 29% sensitivity and a 96.7% specificity to detect dependency at three months with a positive predictive value 90% and a negative predictive value of 56.9%. All these values are shown in Table 4. The combination of both endostatin >184 ng/ml + FasL > 37,1 ng/ml had a specificity of 100% to predict bad outcome.

In the scope of the present application, the "Positive Predictive Value" (PPV) is the proportion of patients with positive test results who are correctly diagnosed; currently those patients whose endostatin plasma level is greater than 184ng/ml and FasL level greater than 37.1 ng/ml, meaning that were dependents (mRS> 2) at three months. "Negative Predictive Value" (NPV) is the proportion of patients with negative test results who are correctly diagnosed; currently those patients who had lower levels of endostatin and FasL levels and were independent (mRS≤2) at three months. Table 4 shows the sensitivity, specificity, PPV and NPV of baseline endostatin and FasL and the combination of both biomarkers in the actual population.

### Table 4

|  | Sensitivity | Specificity | PPV | NPV |
|---|---|---|---|---|
| **FasL 37.1** | 29 | 96.7 | 90 | 56.9 |
| **Endostatin 184** | 45.2 | 86.7 | 77.8 | 60.5 |
| **FasL + Endostatin** | 9 | 100 | 100 | 51.7 |

### Example 4: Synergistic effect of endostatin and FasL

Different clinical variables such as NIHSS, age, type of occlusion were strongly associated with the patient outcome at three months, meaning that older patients with a greater neurological deficit and a baseline proximal occlusion had had a worse outcome at three months. The addition of prognosis biomarkers should improve the utility of clinical predictive models.

In the scope of the present application, the term "odds ratio" (OR) refers to the probability of a certain event was the same for two groups (independent or dependent stroke patients at three months). An odds ratio of 1 implied that the event is equally likely in both groups. An odds ratio greater than 1 implied a higher probability to be functional dependent at three months. The OR and 95% Confidence Intervals (CI) for the effect on dependency at three months were estimated using logistic regression analysis adjusted for the effects of conventional risk factors. The additional contribution of endostatin+FasL biomarkers that were significantly associated with poor outcome after adjustment to the previously validated clinical variable model, is assessed. Different logistic regression models are analyzed, adjusted for different clinical variables of adjusted OR of unfavourable outcome at three months for baseline endostatin and FasL levels, as per in Table 5.

**Table 5**

| Model 1 | | | |
|---|---|---|---|
| **Including NIHSS, age and individual biomarkers** | **OR** | **CI** | **p** |
| **FasL 37.1** | 14.35 | 1.4-138.8 | 0.021 |
| **Endostatin 184** | 5.62 | 1.3-23.7 | 0.019 |
| **Age** | 1.026 | 0.97-9.08 | 0.345 |
| **NIHSS score** | 1.096 | 0.98-1.23 | 0.126 |
| **Model 2** | | | |
| **Including NIHSS, age and combined biomarkers** | | | |
| **FasL + Endostatin** | 4.86 | 1.86-12.74 | 0.001 |
| **Model 3** | | | |
| **Including NIHSS, age, combined biomarkers and baseline TCD** | | | |
| **FasL + Endostatin** | 5.34 | 1.5-19.3 | 0.011 |
| **TCD (distal vs proximal)** | 5.76 | 1.3-25 | 0.019 |
| **age** | 1.03 | 0.9-1.09 | 0.277 |
| **NIHSS score** | 1.04 | 0.9-1.1 | 0.555 |

In the scope of the present application, the **"diagnosis synergistic effect"** is met when the combined statistical significance of two or several molecules is greater than the sum of the separate molecules independently. All statistical analysis was conducted using SPSS 15.0 (SPSS Inc. Chicago, IL, USA). P< 0.05 was considered statistically significant. A lower p value implies stronger statistically significant differences.

Results of the invention showed endostatin level >184 ng/ml (p=0.006) and FasL > 37.1 (p=0.016) were the main baseline predictors of dependency at three months as per in the Model 1 shown in Table 5. After adjustment of age and NIHSS scores, the combination of both biomarkers remained independent as per in Model 2 and the combination showed a lower p value (p=0.001) than each biomarker independently (Table 5). Interestingly, the synergistic effect of both biomarkers was observed in Model 3 because including also in the model the baseline TCD (proximal), the combination endostatin+FasL, was the main independent predictor of dependency at three months. In model 2 and 3 the single scores of the biomarkers did not remain independent and the combination endostatin+FasL was the main predictor of dependency.

The inventors also confirmed that the combination endostatin+FasL was specific. They analyzed stromal derived factor 1 (SDF-1) levels, a biomarker related to angiogenesis process (a process totally different from apoptosis) and tumor necrosis factor receptor 1 (TNFR-1), a biomarker also involved in apoptosis processes. Both biomarkers are associated with worse prognosis at three months independently but not in combination with endostatin. Table 6 shows logistic model including different clinical variables of unfavourable outcome at three months for the combination baseline endostatin and SDF-1 levels. SDF-1 levels were associated with dependency (mRS≥2) at three months [OR=13.6 (1.7-108.8);p=0.014], but not the combination endostatin+SDF-1 (p=0.998).

| Table 6 | | | |
|---|---|---|---|
| **Including NIHSS, age, baseline tcd** | **OR** | **CI** | **p** |
| **SDF-1** | 13.6 | 1.7-108.8 | 0.014 |
| **TCD** | 1.9 | 0.2-16 | 0.553 |
| **Endostatin+SDF-1** | | | 0.998 |

Interestingly, the results also showed an association between TNFR-1 and bad outcome [OR=5.8(1.4-23.1); p=0.012], but not at the' analysis of the combination endostatin+TNFR1 (p=0.998), as per in Table 7, which shows the logistic model including different clinical variables of adjusted OR of unfavourable outcome at three months for baseline endostatin and TNFR-1 levels.

**Table 7**

| Including NIHSS, age, baseline tcd | OR | CI | p |
|---|---|---|---|
| TNFR-1 | 5.8 | 1.4-23.1 | 0.012 |
| TCD | 6.9 | 1.9-25.2 | 0.003 |
| Endostatin+TNFR-1 | | | 0.998 |

In order to compare the ability of models to discriminate between good and poor outcome, the inventors calculated areas under receiver operator curves (AUC). An AUC of 1 indicated perfect discrimination; whereas 0.5, no discrimination. The inventors calculated the prognostic models using clinical variables or clinical variables plus biomarkers.

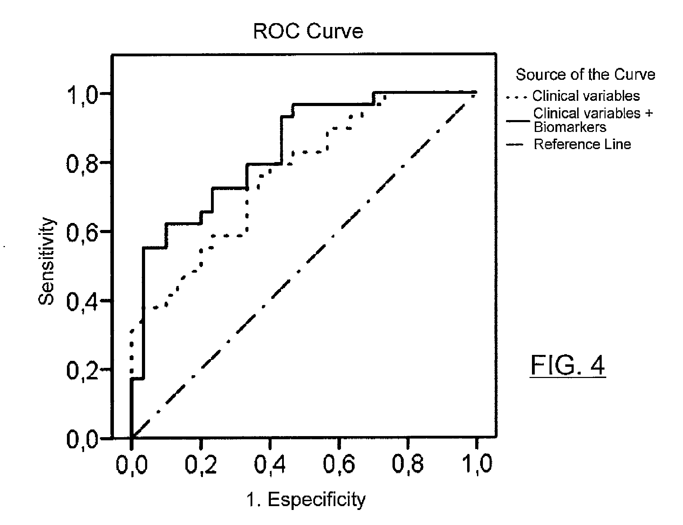

The synergistic effect was also observed in the analysis of the contribution of endostatin+FasL to clinical prognostic models. The addition of biomarkers (AUC value=0.834 vs AUC value=0.766) improved the prediction of poor outcome after stroke (Figure 4). Therefore, the inventors also determined a predictive model that includes clinical variables (baselineTCD) and endostatin+FasL levels to predict outcome. An equation from the regression model was performed to calculate the predictive probability of bad outcome at three months. The probability predicted for dependency (mRS> 2) was obtained from the following equation:

$$\text{Probability (\%)} = \frac{1}{1+e^{-(-9.8 + (1.67^* \text{ Endostatin+FasL}) + (1.75^* \text{ baseline TCD}))}}$$

Biomarkers and clinical variables included in the equation:

Endostatin + FasL (range value 2-5) meaning:

2 : Endostatin< 184 ng/mL and FasL< 37.1 ng/mL
3 : Endostatin ≥184 ng/mL and FasL< 37.1 ng/mL
4 : Endostatin < 184 ng/mL and FasL ≥37.1 ng/mL
5 : Endostatin ≥184 ng/mL and FasL ≥37.1 ng/mL

Baseline TCD (range value 0-2) meaning:

0 : normal occlusion
1: distal occlusion
2: proximal occlusion

**Claims**

1.  Method of predicting the evolution of a patient suffering of a neurovascular disease, comprising:

    a) obtaining a biological sample of said patient;
    b) assessing in said biological sample the level of endostatin and FasL, or of respective nucleic acid molecules encoding same;
    c) determinining whether said levels of endostatin and FasL together, or of respective nucleic acid molecules encoding same, are above or below predetermined cut-off levels; and
    d) predicting the functional outcome of said neurovascular disease on said patient evaluating the result of step c).

2.  A method according to claim 1, **characterized in that** said neurovascular disease is stroke.

3.  A method according to claims 1 or 2, **characterized in that** said biological sample has been taken out from the patient along the next 24 hours after an acute episode of stroke.

4.  A method according to anyone of claims 1 to 3, **characterized in that** said biological sample is a blood sample.

**5.** A method according to anyone of claims 1 to 3, **characterized in that** said biological sample is a plasma sample.

**6.** A method according to anyone of claims 1 to 3, **characterized in that** said biological sample is a serum sample.

**7.** A method according to anyone of claims 1 to 3, **characterized in that** said biological sample is a saliva sample.

**8.** A method according to anyone of claims 1 to 7, comprising endostatin and FasL in combination with at least another biomarker for the prediction of the evolution of a patient suffering of a neurovascular disease.

**9.** A method according to anyone of claims 1 to 8, comprising endostatin and FasL in combination with at least one clinical parameter for the prediction of the evolution of a patient suffering of a neurovascular disease.

**10.** A method according to anyone of the preceeding claims, **characterized in that** said prediction of the evolution of the patient suffering of a neurovascular disease
* enables a physician to determine the degree and type of therapy recommended to prevent or treat said neurovascular disease.

**11.** Use of endostatin and FasL combined as a predictor of the evolution of a patient suffering of a neurovascular disease.

**12.** Kit for performing the method defined in anyone of claims 1 to 10, comprising appropriate reagents for the quantification of endostatine and FasL in a biological sample of a patient suffering of a neurovascular disease, and enabling the comparison of the combined result of said quantification with predermined cut-off values.

**13.** Kit including at least one container that contains appropriate pharmaceutical formulations for the quantification of endostatin and FasL in a biological sample of a patient suffering of a neurovascular disease, instructions for the use of said formulations and a dispositive for determining whether the result of the quantification of endostatin and FasL combined are above or below predetermined cut-off levels indicative of the likehood of a prognosis of functional outcome of the neurovascular disease in said patient.

**14.** A kit according to claims 12 or 13, **characterized in that** said neurovascular disease is stroke.

**15.** System for the prediction of the evolution of a patient of a neurovascular disease comprising data processing means, said data processing means been configured:

- to assess in said biological sample the level of endostatin and FasL, or of respective nucleic acid molecules encoding same;
- to determine whether said levels of endostatin and FasL together, or of respective nucleic acid molecules encoding same, are above or below predetermined cut-off levels; and
- to predict the functional outcome of the neurovascular disease of the patient evaluating the result of the previous determination

**A** ROC Curve

**FIG. 1**

**B** ROC Curve

mRS<2
□ NO
x YES

_FIG. 2_

Baseline FasL>37,1
ng/ml
□ NO
▨ YES

_FIG. 3_

ROC Curve

Source of the Curve
··· Clinical variables
— Clinical variables +
Biomarkers
— Reference Line

FIG. 4

Europäisches
Patentamt

European
Patent Office

Office européen
des brevets

**EUROPEAN SEARCH REPORT**

Application Number

EP 11 38 2047

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Faina Linkov ET AL: "Reliability of tumor markers, chemokines, and metastasis-related molecules in serum", European. Cytokine Network, 1 March 2009 (2009-03-01), pages 21-26, XP55003807, DOI: 10.1684/ecn.2009.0146 Retrieved from the Internet: URL:http://www.jle.com/e-docs/00/04/48/01/vers_alt/VersionPDF.pdf [retrieved on 2011-07-28] * abstract * | 12-14 | INV. G01N33/68 C12Q1/68 |
| Y | EP 2 166 358 A1 (FUNDACIO INST DE RECERCA DE L [ES]) 24 March 2010 (2010-03-24) * pages 2-4 * | 1-15 | |
| Y | MÍRIAM NAVARRO-SOBRINO ET AL: "A large screening of angiogenesis biomarkers and their association with neurological outcome after ischemic stroke", ATHEROSCLEROSIS, vol. 216, no. 1, 26 January 2011 (2011-01-26), pages 205-211, XP55003801, ISSN: 0021-9150, DOI: 10.1016/j.atherosclerosis.2011.01.030 * abstract * | 1-15 | |
|  | -/-- | | TECHNICAL FIELDS SEARCHED (IPC) G01N C12Q |

The present search report has been drawn up for all claims

| Place of search | Date of completion of the search | Examiner |
|---|---|---|
| The Hague | 1 August 2011 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 11 38 2047

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | J. F. ARENILLAS ET AL: "Angiogenesis in Symptomatic Intracranial Atherosclerosis: Predominance of the Inhibitor Endostatin Is Related to a Greater Extent and Risk of Recurrence", STROKE, vol. 36, no. 1, 1 January 2005 (2005-01-01), pages 92-97, XP55003804, ISSN: 0039-2499, DOI: 10.1161/01.STR.0000149617.65372.5d * abstract * | 1-15 | |

----- 

TECHNICAL FIELDS
SEARCHED      (IPC)

The present search report has been drawn up for all claims

| Place of search | Date of completion of the search | Examiner |
|---|---|---|
| The Hague | 1 August 2011 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 38 2047

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2011

| Patent document cited in search report | | Publication date | Patent family member(s) | | Publication date |
|---|---|---|---|---|---|
| EP 2166358 | A1 | 24-03-2010 | EP | 2338058 A1 | 29-06-2011 |
| | | | WO | 2010031821 A1 | 25-03-2010 |

EPO FORM P0459

For more details about this annex : see Official Journal of the European Patent Office, No. 12/82

**EP 2 492 691 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010031821 A
- WO 2009033406 A1
- WO 2004059293 A2

**Non-patent literature cited in the description**

- **SHENHAR-TSARFATY et al.** Interleukin-6 as an early predictor for one-year survival following an ischaemic stroke/transient ischaemic attack. *Int J Stroke,* February 2010, vol. 5 (1), 16-20
- **WHITELEY W et al.** Blood markers for the prognosis of ischemic stroke: a systematic review. *Stroke,* May 2009, vol. 40 (5), e380-9
- **MONTANER J et al.** Etiologic diagnosis of ischemic stroke subtypes with plasma, biomarkers. *Stroke,* August 2008, vol. 39 (8), 2280-7
- **SERENA J et al.** The prediction of malignant cerebral infarction by molecular brain barrier disruption markers. *Stroke,* September 2005, vol. 36 (9), 1921-6
- **MONTANER J.** Blood biomarkers to guide stroke thrombolysis. *Front Biosci (Elite Ed,* 01 June 2009, vol. 1, 200-8
- **TARKOWSKI E et al.** Intrathecal expression of proteins regulating apoptosis in acute stroke. *Stroke,* February 1999, vol. 30 (2), 321-7
- **DELGADO et al.** Fas system activation in perihematomal areas after spontaneous intracerebral hemorrhage. *Stroke,* 2008, vol. 39, 1730-1734
- **ARENILLAS et al.** Angiogenesis in symptomatic intracranial atherosclerosis: predominance of the inhibitor endostatin is related to a greater extent and risk of recurrence. *Stroke,* January 2005, vol. 36 (1), 92-7
- **TIAN et al.** Increased protein and mRNA expression of endostatin in the ischemic brain tissue of rabbits after middle cerebral artery occlusion. *Neurosci Bull.,* 2007, vol. 23 (1), 35-40
- **MONTANER et al.** *High level of an Angiogenesis Inhibitor (endostatin) is Related to poor Outcome in Ischemic Stroke Patients treated with t-PA,* 2005, www.tesisenxarxa.net
- **FOERCH C et al.** Blood biomarkers in acute stroke. *Neurology,* 04 August 2009, vol. 73 (5), 393-9
- **LASKOWITZ DT et al.** *Stroke,* January 2009, vol. 40 (1), 77-85
- **HACKE et al.** Thrombolysis with alteplase 3 to 4.5 hours after acute ischemic stroke. *N Engl J Med.,* 2008, vol. 359, 1317-29
- **BURGIN et al.** Transcranial doppler ultrasound criteria for recanalization after thrombolysis for middle cerebral artery stroke. *Stroke,* 2000, vol. 31, 1128-32